# EUROPEAN PATENT APPLICATION

(11) **EP 0 972 843 A1**
(43) Date of publication of application: **19.01.2000**
(21) Application number: 99113392.7
(22) Date of filing: 10.07.1999
(51) Int. Cl.: C12P 7/60

(54) **Continuous fermentation process**

(30) Priority: 17.07.1998 EP 98113373
(71) Applicant: F. HOFFMANN-LA ROCHE AG, 4070 Basel (CH)
(72) Inventor: Rueckel, Markus, 82377 Penzberg (DE)

(57) **Abstract**

A process for the continuous production of 2-keto-L-gulonic acid or a salt thereof from D-sorbitol by fermentation with microorganisms is characterized in that a nutrient medium containing D-sorbitol is incubated in a first fermentation vessel with a microorganism capable of converting D-sorbitol to L-sorbose, whereafter the resulting fermentation broth containing L-sorbose is transferred to a second fermentation vessel where it is incubated with a microorganism capable of converting L-sorbose to 2-keto-L-gulonic acid. In a particularly preferred embodiment of this process the fermentation broth from the first fermentation vessel is sterilized before being transferred to the second fermentation vessel. 2-keto-L-gulonic acid is a valuable intermediate for the production of vitamin C.

## Description

The present invention relates to a continuous fermentation process for the manufacture of 2-keto-L-gulonic acid (2-KGA) from D-sorbitol. 2-KGA is a valuable intermediate for the production of ascorbic acid (vitamin C).

Processes for the manufacture of 2-KGA from D-sorbitol are known. For example, EP 0 518 136 A2 discloses a fermentation process utilizing a mixed microorganism culture whereby D-sorbitol is oxidized to L-sorbose with a microorganism belonging to the genus Gluconobacter or Acetobacter, e.g., Gluconobacter suboxydans IFO 3291, and the L-sorbose is in turn converted to 2-KGA by fermentation with the microorganism strain DSM 4025, also known as Gluconobacter oxydans DSM 4025. In this process both microorganisms coexist in the fermentation medium during at least part of the entire cultivation period. It has been found, however, that the yield in the process disclosed in EP 0 518 136 A2 is unsatisfactory when the process is carried out in continuous manner.

It is an object of the present invention to provide an efficient process for the manufacture of 2-KGA or a salt thereof from D-sorbitol that produces high yields, minimizes losses from the formation of unwanted by-products, carbon dioxide and cell mass, and can be performed over an extended period of time without interruption, i.e. continously.

Accordingly, the present invention provides a process for the continuous production of 2-KGA or a salt thereof from D-sorbitol by fermentation with microorganisms, in which process a nutrient medium containing D-sorbitol is incubated in a first fermentation vessel with a microorganism capable of converting D-sorbitol to L-sorbose, whereafter the resulting fermentation broth containing L-sorbose is transferred to a second fermentation vessel where it is incubated with a microorganism capable of converting L-sorbose to 2-KGA.

Examples of a microorganisms capable of converting D-sorbitol to L-sorbose are microorganisms of the genera Gluconobacter and Acetobacter, such as
Gluconobacter suboxydans IFO 3130, IFO 3255, IFO 3256, IFO 3257, IFO 3258, IFO 3289, IFO 3290 and IFO 3291;
Gluconobacter gluconicus IFO 3171, IFO 3285 and IFO 3286;
Gluconobacter rubiginosus IFO 3244;
Gluconobacter albidus IFO 3251 and IFO 3253;
Gluconobacter industrius IFO 3261;
Gluconobacter cerinus IFO 3262, IFO 3263, IFO 3265, IFO 3266, IFO 3267 and IFO 3270;
Gluconobacter diacetonicus IFO 3273;
Gluconobacter roseus IFO 3990;
Acetobacter aceti subsp. orleans IFO 3259;
Acetobacter aceti subsp. aceti IFO 3281;
Acetobacter liquefaciens IFO 12257, IFO 12258 and IFO 12388; and
Acetobacter aceti subsp. xylinum IFO 3288, IFO 13693, IFO 13772 and IFO 13773.

Preferred microorganisms are Gluconobacter suboxydans IFO 3255, 3256, 3258, 3290 and 3291; Gluconobacter gluconicus IFO 3285; and Gluconobacter cerinus IFO 3267. The most preferred microorganism is Gluconobacter suboxydans IFO 3291.

The above-named microorganisms are preserved in the public microorganism depositary (culture collection) The Institute of Fermentation Osaka, Japan (IFO) and are available to anyone upon request and payment of the requested fee.

The preferred microorganism for the conversion of L-sorbose to 2-KGA is Gluconobacter oxydans DSM 4025. This strain was deposited on March 17, 1987 at the Deutsche Sammlung von Mikroorganismen in Göttingen, Germany (now located in Braunschweig), based on the stipulations of the Budapest Treaty, under DSM No. 4025. The depositor was The Oriental Scientific Instruments Import and Export Corporation for Institute of Microbiology, Academia Sinica, 52 San-Li-He Rd., Beijing, Peoples Republic of China. The effective depositor was said Institute, of which the full address is The Institute of Microbiology, Academy of Sciences of China, Haidian, Zhongguancun, Beijing 100080, People's Republic of China.

As regards the nutrient medium suitable for the cultivation of the microorganisms used in the process of the invention, although no special restrictions are imposed, an aqueous nutrient medium may include carbon sources and nitrogen sources. Other inorganic salts, small amounts of other nutrients and the like, which can be utilized by the microorganisms are desirable for the advantageous incubation of the microorganisms. Various nutrient materials which are generally used for the better growth of microorganisms may suitably be included in the medium.

In addition to the D-sorbitol which is used as the starting material in the process of the present invention, other substances which are carbon sources may also be present in the nutrient medium, such as glycerol, D-glucose, D-mannitol, D-fructose, D-arabitol and the like.

Various organic or inorganic substances may also be used as nitrogen sources in the process, such as meat extract, peptone, casein, corn steep liquor, urea, amino acids, nitrates, ammonium salts and the like. Magnesium sulfate, potassium phosphate, ferrous and ferric chlorides, calcium carbonate and the like may be used as inorganic substances.

The mixing ratio of these nutrients and the amounts of each ingredient may vary with the generic properties of the microorganisms employed, the amounts of the starting material, D-sorbitol, the amount of one of the microorganisms to be inoculated with respect to the other and the times of inoculations, and the other conditions of the incubation may be selected or determined in accordance with the particulars of the individual case.

The suitable concentration of the starting material, D-sorbitol, in the medium contained in the first fermentation vessel depends on the generic character and the like of the employed microorganism in any instance. In a preferred embodiment, the concentration of D-sorbitol in the nutrient medium in the first fermentation vessel is from about 10 to about 400 g/l , more preferably from about 150 to about 350 g/l. The concentration of L-sorbose, produced in the first formentation vessel, in the fermentation broth fed to the second fermentation vessel is preferably from about 5 to about 200 g/l, more preferably from about 60 to about 180 g/l.

The further conditions of the fermentation (cultivation) may also vary depending on the species and generic character of the particular microorganisms employed The composition of the medium may, of course, be selected or determined in accordance with the particulars of the individual case in order to yield the intended product most efficiently. In the first fermentation vessel the cultivation temperatures are suitably from about 12 to about 38°C, preferably from about 18 to about 32°C, and in the second fermentation vessel the cultivation temperatures are suitably from about 20 to about 33°C. In the first fermentation vessel the pH value of the medium is suitably from about 2.0 to about 9.0, preferably from about 3.0 to about 7.0. In the second fermentation vessel the pH of the broth is suitably from about 5.0 to about 9.0, preferably from about 6.0 to about 8.0.

In a further preferred aspect of the process of the invention the fermentation is carried out at a dissolved oxygen concentration of from about 0.1 to about 200% air saturation, preferably of from about 5 to about 100% air saturation, in the first fermentation vessel; and of from about 8 to about 80% air saturation in the second fermentation vessel.

Furthermore, the fermentation is preferably carried out at oxygen concentrations in the gassing flow of from about 0.1 to about 100%, preferably of from about 15 to about 100%, in the first fermentation vessel, and of from about 19 to about 100% in the second fermentation vessel; and at gassing rates of from about 0.01 to about 1.2 v./v./min. (volume of gas per volume of reactor per minute) in the first fermentation vessel, and of from about 0.03 to about 0.85 v./v./min. in the second fermentation vessel.

It is also preferred to carry out the fermentation in the first fermentation vessel at dilution rates of liquid flow of about 0.02 to about 0.5 h⁻¹, more preferably of about 0.06 to about 0.3 h⁻¹. The dilution rate of liquid flow in the second fermentation vessel is preferably about 0.03 to about 0.25 h⁻¹.

While the fermentation according to the present invention may be carried out at normal pressure, i.e. at about 1 bar, it is generally preferred to work under elevated pressure, e.g. a pressure of at least 3 bar, most preferably at least 5 bar.

In a particular preferred embodiment of the process of the present invention the fermentation broth from the first fermentation vessel is sterilized before being transferred to the second fermentation vessel. This can be achieved by heating the fermentation broth in the first fermentation vessel before transfer to the second to a temperature of about 35 to about 121 °C, preferably of from 45 to 80 °C, or by effecting the heating to such a temperature after the fermentation broth has been removed (discharged) from the first fermentation vessel but before it has been introduced into the second, i.e. during the passage from the first to the second fermentation vessel.

In a further particularly preferred embodiment of the process of the present invention nutrient medium containing D-sorbitol is fed to the first fermentation vessel at a rate higher than the rate at which the fermentation broth containing L-sorbose is transferred from the first fermentation vessel to the second fermentation vessel, while simultaneously part of the fermentation broth containing the produced L-sorbose is discharged from the first fermentation vessel into a separate vessel, thus keeping the volume of fermentation broth in the first fermentation vessel substantially constant.

In another particularly preferred embodiment of the process of the present invention nutrient medium is fed into the second fermentation vessel during the fermentation process. The rate of supply of nutrient medium to the second fermentation vessel is adjusted to the rate of supply of fermentation broth from the first fermentation vessel and to the rate of discharge of fermentation broth from the second fermentation vessel, so that the working volume in the second fermentation vessel is kept substantially constant. The nutrient medium supplied to the second fermentation vessel is suitably substantially the same as the nutrient medium used in the first fermentation vessel except that it contains no sorbitol. In a preferred embodiment, nutrient media of different composition in respect of the nutrients and their concentration are supplied from separate storage containers to the second fermentation vessel at variable rates in order to enable the establishment of optimal conditions for the growth of the microorganism in the fermentation broth.

In order to optimize the yield of the process the fermentation can be carried out by using more than one fermentation vessel in each step of the entire process. For example, the fermentation of D-sorbitol to L-sorbose and/or the fermentation of L-sorbose to 2-KGA can be carried out in two or more fermentation vessels which are positioned in consecutive or parallel order.

Suitably, the microorganism in each fermentation vessel is partially or totally immobilized by methods known per se, such as chemical bonding, e.g., covalent or ionic bonding, crosslinking with polymers, or physical methods for cell retention, e.g., adsorptive bonding, matrix entrappment, microencapsulation or by use of membrane reactors, or combination of such immobilization methods. In a preferred embodiment, the microorganisms are immobilized by cell adhesion to porous organic carriers, e.g. polymers such as cellulose; or inorganic carriers, e.g. minerals such as bentonite or steatite, ceramics or glass beads.

In order to maintain the pH value of the medium to that most suitable for the enzymatic activity, any suitable acidic or basic agent may be added to the medium in a suitable amount at a suitable time during the cultivation. The same object may alternatively be accomplished by initially incorporating a suitable buffer or buffering agent into the medium at the beginning of the cultivation.

The 2-keto-L-gulonic acid thus produced in the second fermentation vessel may be separated and purified by conventional methods known per se, and it may be separated as a salt , e.g. of sodium, potassium, calcium, ammonium or the like if the corresponding metal ions are present in the fermentation (nutrient) medium; this is the case when inorganic salts of sodium, potassium, calcium, ammonium etc. are present. The salt may be converted into the free acid by conventional methods known per se.

The invention is illustrated further by the following Examples, in which the percentages are expressed on the basis of weight/volume.

### Example 1

### Preparation of seed cultures:

(a) An aqueous seed medium for use in the first fermentation vessel containing 10% of D-sorbitol, 0.006% of yeast extract, 0.02% of magnesium sulfate heptahydrate, 1% of corn steep, 0.03% of potassium dihydrogen phosphate and 0.06% of calcium carbonate was prepared.
   One loopful of Gluconobacter suboxydans IFO 3291 was transferred into 300 ml of the seed medium in a 1 l shaking flask. The flask was incubated at 28°C with shaking for 2 days.
(b) An aqueous seed medium for use in the second fermentation vessel containing 4% of L-sorbose, 0.5% of urea, 0.05% of glyceride, 0.25% of magnesium sulfate heptahydrate, 1.75% of corn steep, 5% of yeast extract and 1.5% of calcium carbonate was prepared. Five loopfuls of Gluconobacter oxydans DSM 4025 were transferred into 300 ml of the seed medium in a 1 l shaking flask. The flask was incubated at 28°C with shaking for 3 days.

### Example 2

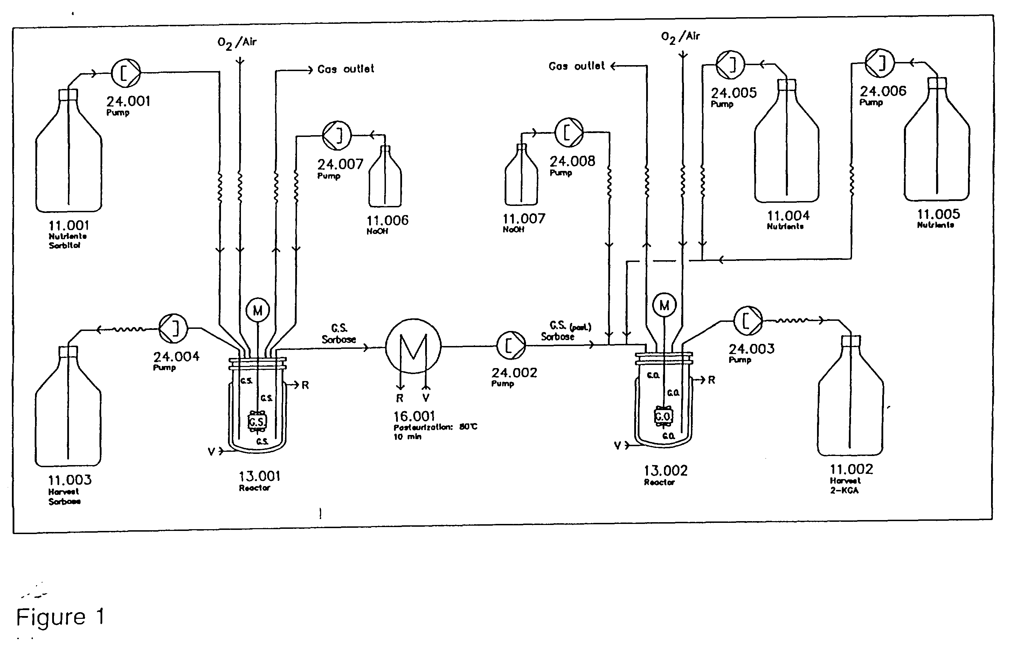

A bioreactor system as shown in Fig. 1 is assembled. The system has two fermentation vessels (reactor 13.001 (first fermentation vessel) and 13.002 (second fermentation vessel)), storage bottles 11.001, 11.004 and 11.005 for nutrient solutions, storage bottles 11.006 and 11.007 for 7.5 M sodium hydroxide solution, harvest bottles 11.002 and 11.003 for harvesting 2-KGA and excess fermentation broth from reactor 13.001 (first fermentation vessel), a loop 16.001 for heating the fermentation broth and pumps 24.001, 24.002, 24.003, 24.004, 24.005, 24.006, 24.007 and 24.008.

Storage bottle 11.001 contains an aqueous solution with 11.5% of D-sorbitol, 1% of corn steep powder, 0.006% of magnesium sulfate heptahydrate, 0.03% of potassium dihydrogen phosphate and 0.06% of calcium carbonate.

Storage bottle 11.004 contains an aqueous solution with 20% of corn steep powder, 0.03% of magnesium sulfate heptahydrate, 0.03% of potassium dihydrogen phosphate and 0.06% of calcium carbonate.

Storage bottle 11.005 contains an aqueous solution with 1% yeast extract, 0.03% of magnesium sulfate heptahydrate, 0.03% of potassium dihydrogen phosphate and 0.06% of calcium carbonate.

The fermentation vessels are equipped with gas supply and a stirrer. A spinning basket is fixed to both stirrer shafts of the fermentation vessels (13.001 and 13.002). The baskets are filled with spherical porous ceramic carrier material having a mean pellet diameter of about 3.5 mm and a mean pore diameter of about 100 micrometer (Ceramtec AG, Marktredwitz, Germany)

### Example 3

The bioreactor system as described in Example 2, with reference to Fig. 1, was put into operation by the following consecutive steps:
1. Assembly of all parts of the reactors, pumps, tubes and instrumentation.
2. Autoclaving at 121°C for 20 minutes in suitable partitions.
3. Preparation of culture broth for continuous operation by dosing the components by weight to the storage bottles and autoclaving (121°C, 20 minutes).
4. Connecting reactors, caustic, storage and harvest bottles under sterile conditions.
5. Charging reactor 13.001 (total volume 2 liters) with 600 ml of the seed medium described in Example 1 (a) under sterile conditions. pH=6.8, temperature =18°C, dissolved oxygen (DO) concentration = 2%ₐᵢᵣ, total pressure (Pₜₒₜ) = 1.1 bar, partial pressure of oxygen (Po₂) = 0.6 bar
6. Caused by oxygen consumption for mainly the conversion of sorbitol to sorbose and the evolution of CO₂ the stirrer speed of the reactor was raised until maintaining a constant value of the DO concentration of 2%. When the sorbitol has been substantially consumed the DO concentration increases due to decrease of oxygen consumption by the microorganism. This triggers the next step:
7. Switching on pump 24.001 for educt supply at 151 ml/h .
8. Charging reactor 13.002 (total volume of 2 liters) with 450 ml of the seed medium described in Example 1 (b) under sterile conditions. pH = 7.0, temperature = 28 °C, DO concentration = 10%ₐᵢᵣ, Pₜₒₜ = 1.1 bar, Po₂ = 0.52 bar.
9. Switching on pump 24.002 for transferring broth from reactor 13.001 to reactor 13.002 at 33 ml/h, pump 24.004 (1.8 ml/h) and pump 24.005 for additional nutrient supply (2 ml/h). The residual sorbose broth was transferred to harvest bottle 11.003. The broth containing 2-keto-L-gulonic acid was harvested in bottle 11.002.
10. Analysis of concentrations of sorbitol, sorbose and 2-keto-L-gulonic acid was effected by high performance liquid chromatography.

Finally the process was operated continuously in stationary phase under the conditions given in the following overview:

**Table 1**

| Process conditions in reactor 13.001 : | | | |
|---|---|---|---|
| pH was controlled with 7.5 M NaOH Total sorbitol concentration (inlet): 113.1 g/kg (119.9 g/l) | | | |
| pH [-] | 6.8 | Sorbose [g/l] | 103.7 |
| T [°C] | 18 | Sorbose [g/kg] | 97.8 |
| DO [%ₐᵢᵣ] | 2 | D [1/h] | 0.126 |
| Pₜₒₜ [bar] | 1.1 | Gassing rate [min⁻¹] | 0.36 |
| Po₂ [bar] | 0.6 | V_{reactor} [l] | 1.2 |
| OD₆₆₀ | 8.45 | CCD [ml⁻¹] | 7.4⁹ |
| D : dilution rate OD : optical density (at 660 nm) CCD : counted cell density Gassing rate : volume of gas stream per reactor volume per minute V_{reactor} : working volume of the reactor | | | |

**Table 2**

| Process conditions in reactor 13.002 : | | | |
|---|---|---|---|
| pH was controlled with 7,5 M NaOH Total C₆ - monosaccharide concentration (inlet): 85.6 g/kg (90.7 g/l) | | | |
| pH [-] | 7.0 | 2-KGA [g/l] | 89.5 |
| T [°C] | 28 | 2-KGA [g/kg] | 84.4 |
| DO [%ₐᵢᵣ] | 10 | D [1/h] | 0.045 |
| Pₜₒₜ [bar] | 1.1 | Gassing rate [vvm] | 0.1 |
| Po₂ [bar] | 0.52 | V_{reactor} [l] | 0.9 |
| OD₆₆₀ [-] | 18.2 | CCD [1/ml] | 2¹⁰ |

### Total system:

Total sorbitol concentration (reactor 13.001): 113.1 g/kg (119.9 g/l)
Dilution factor of broth between reactor 13.001 and reactor 13.002: 0.82

**Table 3**

| Summary results for reactor 13.001 and reactor 13.002: | |
|---|---|
| Overall yield [%_{wt}] | 91 |
| Overall yield [%ₘₒₗ] | 85.5 |
| Productivity [g/l/h]: | 3.1 |

## Claims

1. A process for the continuous production of 2-keto-L-gulonic acid or a salt thereof from D-sorbitol by fermentation with microorganisms, characterized in that a nutrient medium containing D-sorbitol is incubated in a first fermentation vessel with a microorganism capable of converting D-sorbitol to L-sorbose, whereafter the resulting fermentation broth containing L-sorbose is transferred to a second fermentation vessel where it is incubated with a microorganism capable of converting L-sorbose to 2-keto-L-gulonic acid.

2. The process according to claim 1, wherein the fermentation broth from the first fermentation vessel is sterilized before being transferred to the second fermentation vessel.

3. The process according to claim 2, wherein the sterilization is effected by heating the fermentation broth in the first fermentation vessel before being transferred to the second fermentation vessel to a temperature of from about 35 to about 121°C, preferably of from 45 to 80°C, or by effecting the heating to such a temperature after the fermentation broth has been removed from the first fermentation vessel but before it has been introduced into the second.

4. The process according to any one of claims 1 to 3, wherein nutrient medium containing D-sorbitol is fed to the first fermentation vessel at a rate higher than the rate at which the fermentation broth containing L-sorbose is transferred from the first fermentation vessel to the second fermentation vessel, while simultaneously part of the fermentation broth containing the produced L-sorbose is discharged from the first fermentation vessel into a separate vessel, thus keeping the volume of fermentation broth in the first fermentation vessel substantially constant.

5. The process according to any one of claims 1 to 4, wherein nutrient medium is fed into the second fermentation vessel during the fermentation process whereby the supply of nutrient medium to the second fermentation vessel is adjusted to the rate of supply of fermentation broth from the first fermentation vessel and to the rate of discharge of fermentation broth from the second fermentation vessel, so that the working volume in the second fermentation vessel is kept substantially constant.

6. The process according to any one of claims 1 to 5, wherein the microorganism capable of converting D-sorbitol to L-sorbose is a microorganism of the genus Gluconobacter or Acetobacter, preferably Gluconobacter suboxydans IFO 3291.

7. The process according to any one of claims 1 to 6, wherein the microorganism capable of converting L-sorbose to 2-keto-L-gulonic acid is Gluconobacter oxydans DSM 4025.

8. The process according to any one of claims 1 to 7, wherein the concentration of D-sorbitol in the nutrient medium in the first fermentation vessel is from about 10 to about 400 g/l, preferably from about 150 to about 350 g/l.

9. The process according to any one of claims 1 to 8, wherein the concentration of L-sorbose in the fermentation broth fed to the second fermentation vessel is from about 5 to about 200 g/l, preferably from about 60 to about 180 g/l.

10. The process according to any one of claims 1 to 9, wherein the cultivation is carried out in the first fermentation vessel at a temperature of about 12 to about 38°C, preferably from about 18 to about 32 °C, and in the second fermentation vessel at a temperature of from about 20 to about 33 °C.

11. The process according to any one of claims 1 to 10, wherein the cultivation is carried out in the first fermentation vessel at a pH value of the nutrient medium of about 2.0 to about 9.0, preferably at a pH of about 3.0 to about 7.0, and in the second fermentation vessel at a pH value of the broth of from about 5.0 to about 9.0, preferably at a pH of about 6.0 to about 8.0.

12. The process according to any one of claims 1 to 11, wherein the fermentation is carried out at a dissolved oxygen concentration of from about 0.1 to about 200% air saturation, preferably of from about 5 to about 100% air saturation, in the first fermentation vessel, and of from about 8 to about 80% air saturation in the second fermentation vessel.

13. The process according to any one of claims 1 to 12, wherein the fermentation is carried out at oxygen concentrations in the gassing flow of from about 0.1 to about 100%, preferably of from about 15 to about 100%, in the first fermentation vessel, and of from about 19 to about 100% in the second fermentation vessel.

14. The process according to any one of claims 1 to 13, wherein the fermentation is carried out at gassing rates of from about 0.01 to about 1.2 v./v./min. in the first fermentation vessel, and of from about 0.03 to about 0.85 v./v./min. in the second fermentation vessel.
